# EUROPÄISCHE PATENTSCHRIFT

(11) **EP 3 852 838 B1**
(45) Veröffentlichungstag und Bekanntmachung des Hinweises auf die Patenterteilung: **01.05.2024**
(21) Anmeldenummer: 19778889.6
(22) Anmeldetag: 19.09.2019
(51) Int. Cl.: A61M 5/14, A61M 5/50

(54) **VERRIEGELUNG FÜR EIN MODULARES ORDNUNGSSYSTEM**
LOCKING MECHANISM FOR A MODULAR ORGANISATION SYSTEM
DISPOSITIF DE VERROUILLAGE POUR UN SYSTÈME DE RANGEMENT MODULAIRE

(30) Priorität: 19.09.2018 DE 102018122994
(43) Veröffentlichungstag der Anmeldung: 28.07.2021
(73) Patentinhaber: B. Braun Melsungen AG, 34212 Melsungen (DE)
(72) Erfinder: ERLEN, Christoph, 34132 Kassel (DE); GERLACH, Hans-Josef, 34431 Marsberg (DE); SCHÜSSLER, Andreas, 34123 Kassel (DE); SPÖRL, Thomas, 34212 Melsungen (DE)
(74) Vertreter: Winter, Brandl - Partnerschaft mbB
(86) Internationale Anmeldenummer: PCT/EP2019/075217
(87) Internationale Veröffentlichungsnummer: WO 2020/058428

(56) Entgegenhaltungen:
- WO-A1-98/56450
- US-A- 5 836 910
- US-A1- 2015 157 791

## Beschreibung

Die vorliegende Erfindung betrifft ein modulares Ordnungssystem zum Ordnen medizinischer Geräte sowie ein medizinisches Gerät mit einem Gehäuse, welches das modulare Ordnungssystem ausbildet, mit zumindest zwei mechanisch miteinander verbindbaren Ordnungsmodulen, wobei ein erstes Ordnungsmodul einen ersten Gehäuseteil hat, an welchem an zumindest zwei einander diametral gegenüberliegenden Seiten eine erste Koppelstruktur vorgesehen ist, und ein zweites Ordnungsmodul einen zweiten Gehäuseteil hat, welcher dafür angepasst ist, auf, unter oder seitlich an den ersten Gehäuseteil des ersten Ordnungsmoduls montiert/gekoppelt zu werden, und welcher einen drehbar und/oder verschiebbar in dem zweiten Gehäuseteil gelagerten Riegel zum Verriegeln der komplementären Gehäuseteile aufweist.

### Technisches Gebiet

Im medizinisch therapeutischen Umfeld werden häufig mehrere verschiedene Instrumente miteinander verschaltet.

Ein Beispiel hierfür sind Infusionspumpen zur intravenösen Verabreichung von Medikamenten oder Nährstoffen, welche aus Bereichen wie Operationssälen, Intensivstationen, der Onkologie, Schmerztherapie, der klinischen Ernährung, etc. nicht mehr wegzudenken sind. Dabei können sowohl einzelne Infusionspumpen als auch Systeme aus mehreren zusammengeschalteten Infusionspumpen eingesetzt werden, welche für die Infusion verschiedener Stoffe und Dosierungen sowie spezifisch für verschiedene Infusionstherapien eingestellt werden können.

Um ein Verschalten von verschiedenen Geräten, insbesondere Infusionspumpen, zu vereinfachen, existieren diverse Ordnungssysteme/Rack-Systeme. An dieser Stelle setzt die vorliegende Erfindung an. Die vorliegende Offenbarung betrifft modular aufgebaute Ordnungssysteme, um medizinische Geräte, insbesondere Infusionspumpen, mechanisch zu koppeln.

### Stand der Technik

Grundsätzlich ist aus dem Stand der Technik, beispielsweise aus der US 4 756 706 (A), bekannt, Infusionspumpen übereinander an stangen- oder regalartigen Halterungen festzuklemmen oder einzuhängen. Derartige Systeme erlauben es, mehrere Infusionspumpen nahezu beliebig zu kombinieren, hinzuzufügen oder zu entfernen und die kombinierten Pumpen ggf. einfach zu transportieren. Nachteilig ist jedoch, dass derartige Regal- oder Stangenhalterungen einen hohen Raum- und Lageraufwand haben und je nach Beladung instabil werden können. Ferner müssen Klemmen zum Befestigen der Pumpen an der entsprechenden Halterung ggf. hohe Kräfte und Momente halten.

Ferner ist aus der EP 0 780 134 A1 bekannt, mehrere Infusionssysteme direkt aufeinander zu stapeln. Hierbei werden an einer ersten Seite zwei Module derart ineinander eingehakt, dass die Einhakverbindung als ein Scharnier wirkt, um welches die Module zueinander scharnierartig verschwenkt werden können. Anschließend werden sie an einer gegenüberliegenden zweiten Seite verriegelt. Um zwei Module miteinander zu verbinden, müssen diese folglich sorgfältig und somit auch zeitaufwändig platziert werden. Die Verriegelungsstruktur ist relativ komplex und hat ferner eine darin integrierte elektrische Verbindung, weshalb die Reinigung der Bauteile aufwändig ist. Ein Teil der Verriegelung auf der zweiten Seite besteht darüber hinaus aus einem Haken, welcher aufgrund der komplexen Gestaltung der Verriegelung wenig Platz einnehmen darf und sehr schmal ausgeführt ist. Die resultierende Verriegelung ist somit nahezu punktförmig auf eine Ecke der Pumpe begrenzt, was die Stabilität der Verbindung einschränkt. Ferner kann es passieren, dass zwei Module aufeinander gesetzt werden, aber vergessen wird, diese zu verriegeln und somit der Stapel instabil wird, was das sichere Durchführen einer Therapie oder Operation gefährden kann und ferner zu Verletzungen des bedienenden Personals führen kann.

Ebenso sind aus der CN 203 677 636 U stapelbare medizinische Pumpen bekannt, wobei Füße einer ersten Pumpe Schienen bilden, in welche eine Leiste auf der Oberseite einer zweiten Pumpe eingeschoben wird, wodurch ein geschützt zwischen den Pumpen angeordneter, federnd gelagerter Riegel weggedrückt wird, welcher nach Erreichen der Stapelposition einschnappt und somit eine automatische Sicherung darstellt. Dennoch kann es hierbei passieren, dass die Pumpen unbemerkt nicht korrekt gestapelt werden, der Riegel nicht einschnappt und somit die Stapelung instabil ist. Ferner können die Füße sich in den Schienen leicht verklemmen, oder zumindest nicht gleichmäßig und ruckfrei eingeschoben und wieder herausgezogen werden. Bei Lösen der Verbindung muss der Riegel wieder heruntergedrückt und gleichzeitig die Pumpen gegeneinander verschoben werden, was dadurch, dass der Riegel zwischen den Pumpen angeordnet und nicht einfach zugänglich ist, umständlich ist und die Gefahr besteht, dass Personal sich die Hand zwischen den Pumpen einklemmt. Zudem sind die nach innen gewandten, an einer Pumpenunterseite vorgesehenen Schienen ggf. nur schwer zugänglich und ist somit deren Reinigung erschwert. Patent WO 98/56450 A1 offenbart teilweise die Merkmale, die in Anspruch 1 beansprucht sind.

### Zusammenfassung der Erfindung

Die Erfindung wird im Anspruch 1 definiert und weitere Ausführungsformen finden sich in den abhängigen Ansprüchen. Die gegenüber dem Stand der Technik zu lösende Aufgabe besteht somit darin, ein Ordnungssystem für miteinander zu verbindende medizinische Geräte, insbesondere Infusionspumpen, bereitzustellen, welche die vorstehend beschriebenen Nachteile vermeiden. Insbesondere soll ein Ordnungssystem bereitgestellt werden, welches es ermöglicht, mehrere Ordnungsmodule oder Geräte schnell, einfach, intuitiv, sicher und platzsparend mechanisch miteinander zu koppeln.

Die Aufgabe wird insbesondere gelöst durch ein modulares Ordnungssystem zum Ordnen medizinischer Geräte, insbesondere Infusionspumpen, mit zumindest zwei mechanisch miteinander verbindbaren Ordnungsmodulen. Ein erstes Ordnungsmodul hat einen ersten Gehäuseteil, an welchem an zumindest zwei einander diametral gegenüberliegenden Seiten eine erste Koppelstruktur vorgesehen ist. Ein zweites Ordnungsmodul hat einen zweiten Gehäuseteil, welcher dafür angepasst ist, auf, unter oder seitlich an den ersten Gehäuseteil des ersten Ordnungsmoduls montiert zu werden, und welcher einen drehbar und/oder verschiebbar in dem zweiten Gehäuseteil gelagerten Riegel/Joch zum Verriegeln der komplementären Gehäuseteile aufweist. Der Riegel ist derart gestaltet, dass er durch eine singuläre Betätigung in die erste Koppelstruktur an zumindest zwei einander diametral gegenüberliegenden Seiten des ersten Gehäuseteils des ersten Ordnungsmoduls vorzugsweise gleichzeitig hinterschneidend eingreift.

Als singuläre Betätigung ist in diesem Zusammenhang zu verstehen, dass nur eine einzelne Handhabung oder Bewegung benötigt wird, um den Riegel einhändig zu betätigen. Die dabei durchgeführte, einhändige Bewegung ist eine einzelne, zusammenhängende Bewegung und nicht eine Abfolge von Bewegungen. Somit können die Ordnungsmodule des erfindungsgemäßen Ordnungssystems einfach und intuitiv verriegelt werden.

Obwohl das Ordnungssystem vorteilhafter Weise zum Koppeln, insbesondere Stapeln, von Infusionspumpen vorgesehen ist, soll dies nicht darauf einschränkend gelten, da es gleichermaßen für eine Vielzahl anderer Geräte oder Gerätekombinationen eingesetzt werden kann, die Rack-artig angeordnet sein sollen.

Grundsätzlich kann das Ordnungssystem ein Rack bzw. eine Regalstruktur sein, mittels welcher eine Anzahl medizinischer Geräte mechanisch und ggf. elektrisch gekoppelt werden können, vorteilhafterweise als ein platzsparender, ergonomisch zugänglicher Geräteturm aus senkrecht aufeinander gestapelten und verriegelten Geräten. Dabei kann jedes Ordnungsmodul als ein Fach angesehen werden, in welches ein oder mehrere Geräte eingesetzt und angeschlossen werden können. Vorteilhaft ist es, wenn jedes Ordnungsmodul als Docking-Station für das darin eingesetzte Gerät dient, in welche das Gerät einfach einlegbar ist und beim Einlegen an einer definierten Position die relevanten Kontakte hergestellt werden, ohne dass weitere Anschlüsse manuell hergestellt werden müssen. Darüber hinaus ermöglicht ein derartiges Ordnungssystem es, eine bestimmte Kombination von Geräten für diverse Anwendungen vorzubereiten und einfach und sicher zu transportieren, wobei die Wände des Ordnungssystems ferner als Schutz für die darin eingesetzten Geräte dienen können. Andererseits erlaubt dieses Ordnungssystem es, einzelne Pumpen auch in dem fertig vorbereiteten Geräteturm auszutauschen, beispielsweise bei einem Defekt, für Wartungszwecke, oder zum Nachfüllen oder Austauschen von ggf. vorhandenen Behältern.

Alternativ zu der vorstehend beschriebenen regalartigen Ausbildung des Ordnungssystems können die Ordnungsmodule direkt an die zu koppelnden Geräte angeschlossen sein. D.h., die Gehäuseteile der Ordnungsmodule sind gleichzeitig Teil der Gehäuse der zu koppelnden Geräte, oder sind alternativ dafür angepasst, an den Geräten befestigt zu werden, sodass ein Herausnehmen eines Gerätes aus dem Geräteturm nur möglich ist, wenn das gesamte zugehörige Ordnungsmodul herausgenommen wird.

An einzelnen oder sämtlichen Ordnungsmodulen können Halterungen oder Fächer für Zubehör, wie beispielsweise Medikamentenbeutel oder Messgeräte, für die zu koppelnden oder gekoppelten Geräte vorgesehen sein. Ferner können die Ordnungsmodule identisch zueinander sein oder hinsichtlich spezifischer Funktionen spezialisiert ausgebildet sein, solange Kopplungsseiten, an der die beiden Ordnungsmodule miteinander verbunden werden bzw. sind, zueinander kompatibel sind. An den Kopplungsseiten der Module können ferner zueinander kompatible elektronische Schnittstellen für Daten- und/oder Energieübertragung vorgesehen sein.

Die erste Koppelstruktur des einen Ordnungsmoduls ist dafür vorgesehen, in hinterschneidenden Eingriff mit dem Riegel des anderen Ordnungsmoduls zu kommen. Beispielsweise ist die erste Koppelstruktur als ein Vorsprung oder mehrere Vorsprünge wie Haken oder Rippen oder durch eine Aussparung oder mehrere Aussparungen wie Nuten, Spalten oder Löcher ausgebildet. Während einer Verriegelungsbewegung greift der Riegel hinter die erste Koppelstruktur, wobei eine Anlagefläche des Riegels an einer Anlagefläche der Koppelstruktur in Anlage kommt. Dies verhindert, dass die beiden Ordnungsmodule sich in einem verriegelten Zustand voneinander weg bewegen können, zumindest in einer Richtung senkrecht zu den aneinander anliegenden Anlageflächen. Im Beispiel des vorstehend genannten Geräteturms ist die Verriegelung dazu konfiguriert, eine Bewegung der einzelnen Ordnungsmodule in einer Höhenrichtung voneinander weg zu unterbinden. Der Riegel kann sowohl durch Verschwenken um eine Drehachse als auch durch Verschieben in einer Verriegelungsrichtung oder durch eine kombinierte Schwenk-Linearbewegung in eine Verriegelungsposition bzw. -stellung gebracht werden. Der Riegel kann beispielsweise als ein oder mehrere miteinander gekoppelte Bolzen, Balken oder Haken, ausgebildet sein. Alternativ oder zusätzlich kann der Riegel in eine Richtung manuell betätigbar sein und in der anderen Richtung durch eine Federkraft betätigbar sein.

Erfindungsgemäß greift der Riegel an zumindest zwei einander diametral gegenüberliegenden Seiten des ersten Gehäuseteils in die erste Koppelstruktur ein. Auf diese Weise kann sichergestellt werden, dass die beiden Gehäuseteile vollständig miteinander verriegelt werden und Verkippungsmomente zwischen den beiden Ordnungsmodulen sicher gehalten werden. Diametral einander gegenüberliegend sind zwei Positionen eines Ordnungsmoduls, welche einander über dessen Symmetrieachse oder -ebenen hinweg gegenüberliegen, und im Falle eines rechteckigen Moduls insbesondere zwei oder vier einander über dessen Symmetrieachse hinweg gegenüber liegende Ecken oder Seitenmittelpunkte. Die Positionen können an der Kopplungsseite liegen oder unmittelbarer neben der Kopplungsseite an daran anschließenden Seitenflächen der Ordnungsmodule.

Werden die beiden Ordnungsmodule gemäß dem Beispiel des Geräteturms aufeinander montiert, hilft vorzugsweise das Eigengewicht des oberen Ordnungsmoduls, diese zu fügen. Insbesondere können die Gehäuseteile zueinander komplementäre, beim Fügen/Montieren ineinandergreifende Strukturen haben, welche eine Relativbewegung der Ordnungsmodule in andere Richtungen als der, in welche die Verriegelung wirkt, (d.h., im Beispiel des Geräteturms in seitliche Richtungen) unterbinden, was ebenfalls durch das Eigengewicht des oberen Moduls begünstigt wird.

Vorteilhafte Ausführungsformen sind in den Unteransprüchen beansprucht und werden nachfolgend erläutert.

Vorzugsweise hat jedes der zumindest zwei Ordnungsmodule sowohl den ersten als auch den zweiten Gehäuseteil und ist jedes Ordnungsmodul mit jedem beliebigen weiteren Ordnungsmodul koppelbar. Auf diese Weise können die Ordnungsmodule in beliebiger Reihenfolge und nahezu unbegrenzter Anzahl gestapelt werden.

Vorzugsweise hat die erste Koppelstruktur ein Raster von ersten Vorsprüngen und hat der Riegel eine zweite Koppelstruktur mit einem komplementären Raster von zweiten Vorsprüngen, welche dafür angepasst sind, in die ersten Vorsprünge hinterschneidend einzugreifen.

Auf diese Weise sind die Kopplungsstrukturen gleichmäßig über die zur Verfügung stehende Fläche verteilt, insbesondere die erste Koppelstruktur entlang von zwei Seitenrändern der Kopplungsseite des ersten Ordnungsmoduls und die zweite Koppelstruktur entlang von zwei Seitenrändern des Riegels, was eine nahezu ideal verteile Kraft- und Momentenübertragung (insbesondere des Verkippungsmoments) ermöglicht. Vorzugsweise sind mindestens 2 mal 2 zweite Vorsprünge vohanden oder eine an die Größe der zur Verfügung stehenden Fläche und an die erwarteten mechanischen Belastungen angepasste Anzahl, z.B. 2 mal 9, zweite Vorsprünge vorhanden. Somit müssen die einzelnen Vorsprünge nicht weit vorspringen und der Riegel nicht übermäßig dick in Höhenrichtung sein, um die benötigte Stabilität zu erzielen. Die Verriegelung der beiden Ordnungsmodule kann somit hohen mechanischen Kräften standhalten. Der Riegel kann aus einem Metall, z.B. Edelstahl, oder einem ggf. glasfaserverstärkten technischen Kunststoff, z.B. POM oder PEI, gefertigt sein, welcher die für die Anwendung notwendige Festigkeit, Reinigungs- und Desinfektionsmittelbeständigkeit aufweist. Aufgrund der einfachen Geometrie des Riegels ist dieser einfach herzustellen und zu reinigen.

Die ersten Vorsprünge sind vorzugsweise nebeneinander angeordnet, d.h. in zumindest einer Reihe an zumindest zwei einander diametral gegenüberliegenden Seiten der Kopplungsseite. Dementsprechend sind die zweiten Vorsprünge in diesem Fall ebenfalls nebeneinander in zumindest einer Reihe an zu den ersten Vorsprüngen passenden Positionen angeordnet. Dabei haben die ersten und zweiten Vorsprünge in ihrer Anordnungsrichtung eine bestimmte Maximalbreite und einen bestimmten Minimalabstand zueinander. Die erste und zweite Kopplungsstruktur ist also im Wesentlichen kammartig. Diese Maximalbreite und dieser Minimalabstand entsprechen einander und bestimmen ein dem Raster zugrundeliegendes bestimmtes Rastermaß.

Vorzugsweise ist der Riegel, welcher weiter vorzugsweise U-förmig ist, linear um eine bestimmte Distanz, beispielsweise zwischen 3 und 10 mm, vorzugsweise um 5 mm, verschiebbar, wobei das Raster bzw. Rastermaß der ersten und zweiten Vorsprünge die bestimmte Distanz festlegt. Vorteilhafterweise sitzt die erste Koppelstruktur an einem U-förmigen oder O-förmigen, an der Kopplungsseite des ersten Ordnungsmoduls angeordneten Rahmen und die zweite Koppelstruktur an einem U-förmigen oder O-förmigen Riegel, welche ineinander eingeschoben werden und im Falle von U-förmigen Rahmen und Riegeln vorzugsweise einander entgegengesetzt angeordnet sind. Der Rahmen kann in diesem Fall ferner eine Schutzfunktion für die Daten- und/oder Energieübertragungseinheiten erfüllen, wenn diese innerhalb der U-Form oder O-Form angeordnet sind.

Der Riegel ist derart gelagert, dass die bestimmte Distanz eine maximale Verschiebungsdistanz ist, welche dem Rastermaß entspricht. Wenn ausgehend von einer Verriegelungsstellung, in welcher die ersten und zweiten Vorsprünge miteinander fluchten, der Riegel um die bestimmte Distanz verschoben bzw. entriegelt wird, werden die ersten und zweiten Vorsprünge in eine zueinander versetzte Lage verschoben, was einer Entriegelungsstellung entspricht. In dieser Lage können die ersten und zweiten Vorsprünge kammartig zwischen einander durch bewegt werden und können somit die beiden Ordnungsmodule in einer Richtung senkrecht zu der Kopplungsseite und zu der Ver- bzw. Entriegelungsrichtung voneinander getrennt werden.

Vorzugsweise haben die ersten Vorsprünge der ersten Koppelstruktur des Rahmens in eine Entriegelungsrichtung des Riegels gewandte erste Rampen. Alternativ oder zusätzlich können die zweiten Vorsprünge der zweiten Koppelstruktur des Riegels in eine Verriegelungsrichtung des Riegels gewandte zweite Rampen aufweisen.

Die Rampen liegen an einander zugewandten Seiten der ersten bzw. der zweiten Koppelstruktur, wodurch diese bei einer Bewegung des Riegels in Verriegelungsrichtung ggf. aufeinander treffen, aneinander entlang gleiten und somit sicherstellen, dass der Riegel nicht seitlich gegen die erste Koppelstruktur stoßen und dort blockieren oder verkanten kann. Ferner werden dadurch die beiden Ordnungsmodule zueinander hingezogen. Dies ist insbesondere hinsichtlich ggf. vorhandener elektronischer Schnittstellen wichtig, da diese hierdurch geschlossen werden. Darin, dass bei der Kopplung nach dieser Ausführungsform die beiden Ordnungsmodule ausschließlich in einer zu der Kopplungsseite senkrechten Richtung zueinander relativbewegt werden und nicht verschwenkt oder verdreht werden, wie es beispielsweise bei einer scharnierartigen Anordnung der Fall wäre, liegt ein besonderer Vorteil. Auf diese Weise können auch Schnittstellen eingesetzt werden, welche empfindlich gegenüber Fluchtungsfehlern beim Einstecken sind.

Vorzugsweise hat der Riegel in dessen Verriegelungsrichtung und/oder Entriegelungsrichtung (Verschiebungsrichtungen) jeweils einen Anschlag, welcher dazu angepasst ist, um mit einem zugehörigen Anschlag des zweiten Gehäuseteils in Anlage zu kommen.

Dieser kann beispielsweise in Form eines Vorsprungs oder einer Tasche ausgebildet sein, dessen bzw. deren Enden in Verschiebungsrichtung jeweils als einer der Anschläge dienen. Auf diese Weise kann eine Verschiebung des Riegels begrenzt werden, sodass er beispielsweise nicht über die Ver- bzw. Entriegelungsstellung hinaus verschoben werden kann und/oder nicht aus dem zweiten Ordnungsmodul herausfallen kann.

Vorzugsweise liegt ein eine Markierung aufweisender Teil des Riegels frei, wenn die zwei Ordnungsmodule sich in einem unverriegelten Zustand befinden, um ein auffälliges visuelles Feedback bezüglich des Zustands bereitzustellen. Weiter vorzugweise ist der die Markierung aufweisende Teil des Riegels durch das zweite Gehäuseteil bedeckt, wenn die zwei Ordnungsmodule sich in einem verriegelten Zustand befinden.

Dies ist insbesondere möglich, wenn ein Ende des Riegels in dem Entriegelungszustand aus dem Gehäuse, z.B. aus einem darin vorgesehenen Schlitz, hervorsteht und das entsprechende Ende farbig, vorzugsweise signalfarbig, markiert ist. Beispielsweise können eine Ober- und Unterseite des Riegels vollständig eingefärbt sein, oder kann das Material des Riegels an sich als eingefärbter Kunststoff ausgewählt werden. Wichtig ist lediglich, dass eine Oberfläche des Riegels, welche im Entriegelungszustand freiliegt, an einer für Bedienpersonal auffälligen Stelle befindet, um eine Signalwirkung zu erzeugen und sofort ins Auge zu fallen, falls ein Verriegeln der Ordnungsmodule vergessen wurde. Im Verriegelten Zustand ist der die Markierung aufweisende Teil des Riegels entsprechend verdeckt oder in dem Ordnungsmodul eingeschoben und erzeugt somit keine Signalwirkung. Ferner ist es denkbar, zusätzlich in dem entsprechenden Ende des Riegels eine LED zu integrieren, um die Signalwirkung zu maximieren. Es ist anzumerken, dass die Markierung bei der vorliegenden Erfindung von einer einfachen Beschriftung zu unterscheiden ist. Eine Beschriftung ist beispielsweise in einem Fall vorhanden, in welchem ein mit einem Pfeil oder Ähnlichem versehener Knopf auf von einer mit "offen" beschrifteten Anzeige zu einer mit "geschlossen" gekennzeichneten Anzeige bewegt wird, welche keine der Markierung gemäße Signalwirkung erzeugen kann.

Vorzugsweise ist ein drehbar in dem zweiten Gehäuseteil gelagertes, freiliegendes Riegelschloss zum Betätigen des Riegels vorgesehen. Dabei kann die Drehachse des Riegelschlosses senkrecht zu einer Gehäusewand des zweiten Ordnungsmoduls sein, an welcher das Riegelschloss für einen Benutzer zugänglich ist. Ein drehbares Riegelschloss hat vorteilhafterweise einen geringen Platzbedarf und ist einfach zu bedienen. Vorzugsweise ist das Riegelschloss von durch das Bedienpersonal nur mittels eines einfachen Werkzeugs wie z.B. einer Münze, eines Schraubenziehers oder eines einsteckbaren Hebels betätigbar. Alternativ kann jedoch auch ein fest angebrachtes Betätigungselement wie ein Hebel oder Knopf vorgesehen sein.

Vorzugsweise hat das Riegelschloss in einer Drehrichtung des Riegelschlosses jeweils einen vorwärts und/oder rückwärts gewandten Anschlag, welcher dazu angepasst ist, um mit einem zugehörigen Anschlag des zweiten Gehäuseteils in Anlage zu kommen. Weiter vorzugsweise greift das Riegelschloss in eine Führungsnut des Riegels ein, wobei die Führungsnut derart geformt ist, dass eine Drehbewegung des Riegelschlosses über diese Führungsnut in eine Linearbewegung des Riegels gewandelt wird. Aufgrund der einfachen Geometrie des Riegelschlosses ist dieses einfach herzustellen und zu reinigen.

Die Drehbewegung des Riegelschlosses kann beispielsweise über einen Kontakt mit einer passend geformte Führungsnut oder Stützfläche des Riegels in eine Schwenk- oder Verschiebebewegung des Riegels umgesetzt werden. Alternativ ist auch ein Hebelsystem denkbar.

Die Anschläge des Riegelschlosses können alternativ oder zusätzlich zu den an dem Riegel vorgesehenen Anschlägen vorgesehen sein, um eine Drehbewegung des Riegelschlosses und somit eine Ver- und Entriegelungsbewegung des Riegels zu begrenzen. Insbesondere wenn die Bewegung des Riegelschlosses über einen in einer Führungsnut oder Stützfläche geführten Teil (z.B. ein Stift) des Riegelschlosses übertragen wird, stellen die Anschläge sicher, dass das Riegelschloss nicht überdreht und dieser Kontakt zwischen Riegel und Riegelschloss dadurch gelöst wird (z.B., dass der Stift nicht aus der Führungsnut oder von der Stützfläche rutscht).

Vorzugsweise liegt ein eine Markierung aufweisender Teil des Riegelschlosses frei, wenn die zwei Ordnungsmodule sich in einem unverriegelten Zustand befinden, um ein auffälliges visuelles Feedback bezüglich des Zustands bereitzustellen. Dies ist eine Alternative zu der vorstehend beschriebenen Variante, bei welcher die Markierung an dem Ende des Riegels vorgesehen ist, welche zwar ermöglicht, auf vorstehende Teile zu verzichten, welche vorstehen können und an welchen Bedienpersonal ggf. hängen bleiben kann, welche jedoch auch weniger auffällig und ggf. leicht im Sichtfeld eines unachtsamen Bedieners verdeckt werden kann.

Ferner wird die Aufgabe gelöst durch ein medizinisches Gerät, insbesondere Infusionspumpe, mit einem Gehäuse, welches das vorstehend beschriebene modulare Ordnungssystem ausbildet, wobei vorzugsweise das erste Ordnungsmodul ein Gehäusedeckel und das zweite Ordnungsmodul ein Gehäuseboden ist

### Kurzbeschreibung der Figuren

Fig. 1 zeigt eine perspektivische Ansicht eines ersten Gehäuseteils gemäß einer Ausführungsform der vorliegenden Erfindung;
Fig. 2 zeigt eine perspektivische Ansicht eines Riegels gemäß der Ausführungsform;
Fig. 3A / 3B zeigen zwei perspektivische Ansichten des Riegelschlosses gemäß der Ausführungsform;
Fig. 4 zeigt eine Stellung des Riegels und des ersten Gehäuseteils zueinander in der Verriegelungsstellung und in der Entriegelungsstellung;
Fig. 5 zeigt eine perspektivische Ansicht des zweiten Gehäuseteils gemäß der Ausführungsform;
Fig. 6 zeigt eine teilweise Schnittansicht des ersten Gehäuseteils gemäß der Ausführungsform, wobei die Schnittebene senkrecht zu einer Bewegungsrichtung des Riegels liegt; und
Fig. 7 veranschaulicht einen Montagevorgang zweier Ordnungsmodule gemäß der Ausführungsform.

### Beschreibung der Ausführungsbeispiele

Nachstehend wird ein Ausführungsbeispiel der vorliegenden Offenbarung auf der Basis der zugehörigen Figuren beschrieben. Dabei bezeichnen gleiche Referenzzeichen gleiche Bauteile. Die Erfindung ist nicht durch das Ausführungsbeispiel beschränkt. Andere denkbare Ausführungsformen sowie verschiedene Änderungen und Modifikationen sind in dem durch die Ansprüche definierte Umfang der Erfindung enthalten.

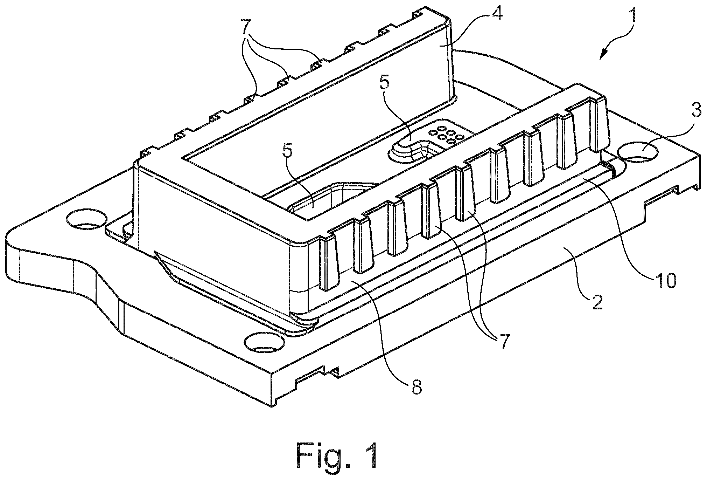

Fig. 1 zeigt eine perspektivische Ansicht eines ersten Gehäuseteils 1, welches in diesem Ausführungsbeispiel ein oberer Gehäuseteil oder Deckel ist. Grundsätzlich kann es jedoch auch ein unterer Gehäuseteil sein. Es weist eine waagrechte, sich parallel zu einer durch ein Koordinatensystem gekennzeichnete x-y-Ebene erstreckende Grundplatte 2 auf, welche Durchgangslöcher 3 aufweist, um mittels einer Schraubverbindung an einem Basisgehäuse eines zugehörigen ersten Ordnungsmoduls O1 montiert zu werden. Anstelle der Schraubverbindung sind alternativ auch andere Befestigungsvarianten wie eine Klipp-, Schweiß oder eine türähnlich Verbindung, bei welcher die Grundplatte an dem Basisgehäuse einseitig angelenkt und anderseitig verriegelt ist, denkbar.

Ein U-förmiger Rahmen 4 ist integral mit der Grundplatte 2 ausgebildet und erstreckt sich senkrecht von der Grundplatte 2 nach oben, in z-Richtung. In dieser Darstellung verläuft eine Basis der U-Form (U-Basis) des Rahmens 4 in einer y-Richtung und verlaufen Schenkel der U-Form (U-Schenkel) des Rahmens 4 in einer x-Richtung. Die U-Form des Rahmens 4 erstreckt sich hierbei nahezu über die gesamte Breite und Länge der Grundplatte 2, weshalb die beiden freien Enden der U-Form des Rahmens 4 sowie die Enden, welche jeweils mit der U-Basis des Rahmens 4 verbunden sind, einander auf der Grundplatte 2 im Wesentlichen diametral gegenüber liegen. Es ist anzumerken, dass nachfolgend die Begriffe "innen" oder "nach innen gewandt" und "außen" oder "nach außen gewandt" im Zusammenhang mit der U-Form sich stets auf das Innere bzw. das Äußere der U-Form beziehen, d.h. den Raum zwischen den U-Schenkeln bzw. den Raum außerhalb der U-Schenkel und der U-Basis betreffen.

Innerhalb der U-Form des Rahmens 4 ist Platz für Daten- und/oder Energieübertragungseinheiten 5 wie elektrische Steckverbindungen vorgesehen. Der Rahmen 4 umgibt die Daten- und/oder Energieübertragungseinheiten 5 und ragt über diese hinaus, wodurch er als Schutzstruktur fungiert. Außenflächen, zumindest in x-Richtung und ggf. auch in y-Richtung gewandte Außenflächen, des Rahmens 4 liegen an komplementären Innenflächen eines später beschriebenen, an einem zweiten Ordnungsmodul O2 befestigten zweiten Gehäuseteils 6 an und bestimmen auf diese Weise die Position der beiden Gehäuseteile 2, 6 zueinander zumindest in x-Richtung, wenn die beiden Ordnungsmodule O1, 02 aneinander montiert sind. Diese Außen- bzw. Innenflächen können komplementär zueinander abgeschrägt sein, sodass ein oberer Rand der Außenflächen bei einer Draufsicht auf den ersten Gehäuseteil 1 weiter als ein unterer Rand der Außenflächen von einem Außenrand der Grundplatte 2 entfernt ist. Auf diese Weise können die Außen- bzw. Innenflächen als Zentrierungselemente dienen, welche ein Aufeinandersetzen und ein genaues Anordnen der beiden Gehäuseteile 2, 6 erleichtern.

Außenseitig an dem U-förmigen Rahmen 4 ist eine erste Koppelstruktur 7 vorgesehen. Diese erste Koppelstruktur 7 weist eine Anzahl von ersten Vorsprüngen oder Rippen auf, welche entlang der U-Schenkel des Rahmens 4 in einem bestimmten Raster mit einem bestimmten Rastermaß vorzugsweise gleichmäßig verteilt sind. Das Rastermaß wird durch eine Breite sowie einen Abstand der Vorsprünge in der x-Richtung definiert. Zwischen der ersten Koppelstruktur 7 und der Grundplatte 2 ist entlang der U-Schenkel des Rahmens 4 ein Spalt 8 ausgebildet, welcher dazu dient, einen Riegel 9, welcher in dem zweiten Gehäuseteil 6 gelagert ist, aufzunehmen. Wenn die beiden Gehäuseteile 1, 6 aufeinander gesetzt sind, ist der Riegel 9 bezüglich beiden Gehäuseteilen 1, 6 in der x-Richtung um eine bestimmte Distanz D (Verschiebedistanz) verschiebbar, wobei die Verschiebung in positiver x-Richtung, d.h. hin zu der Seite der U-Basis des Rahmens 4, einer Verriegelungsrichtung entspricht und die Verschiebung in negativer x-Richtung, d.h. hin zu der Seite der freien Enden der U-Schenkel des Rahmens 4, einer Entriegelungsrichtung entspricht. Die Verschiebungsdistanz D des Riegels 9 zwischen einer Verriegelungsstellung und einer Entriegelungsstellung entspricht dem vorstehend beschriebenen Rastermaß der ersten Koppelstruktur 7. Um den U-förmigen Rahmen 4, insbesondere entlang der U-Schenkel des Rahmens 4, hat die Grundplatte 2 eine integral mit dieser ausgebildete, flache Erhebung 10, auf welcher der Riegel 9 gleitet. Auf diese Weise kann die Größe der für ein flüssiges Gleiten des Riegels 9 nachzubearbeitenden Fläche begrenzt und somit Kosten bei der Herstellung gespart werden.

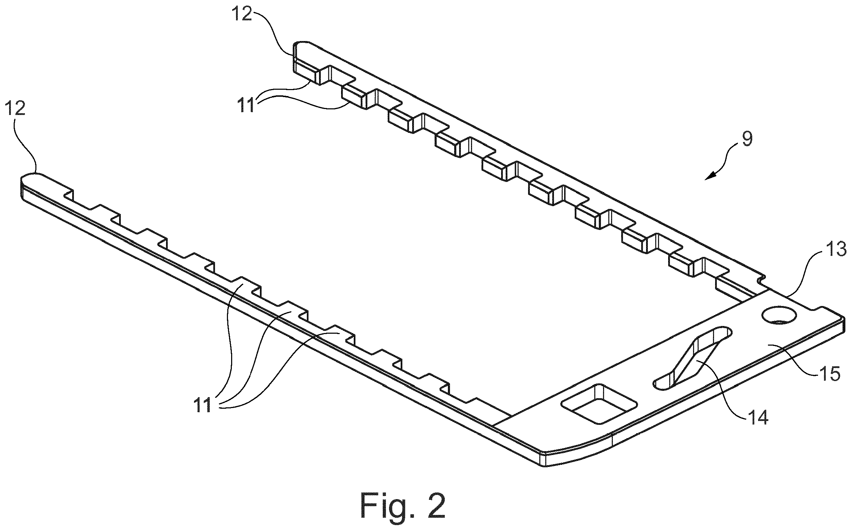

Fig. 2 zeigt eine perspektivische Ansicht des Riegels 9. Dieser weist ebenfalls eine U-Form mit einer in y-Richtung verlaufenden U-Basis und zwei in x-Richtung verlaufenden U-Schenkeln auf, welche U-Form etwas breiter als die U-Form des Rahmens 4 ist, sodass der Riegel 9 über den Rahmen 4 geschoben werden kann. Entlang der U-Schenkel des Riegels 9, dem Inneren der U-Form des Riegels 9 zugewandt, ist eine zu der ersten Koppelstruktur 7 komplementäre zweite Koppelstruktur 11 ausgebildet, welche eine Anzahl von entlang dieser U-Schenkel gleichmäßig verteilter zweiter Vorsprünge oder Rippen aufweist, deren Breite und Abstand durch das vorstehend beschriebene Raster bzw. Rastermaß der ersten Koppelstruktur 7 bestimmt ist. Der Abstand zwischen nach innen gewandten Flanken der U-Schenkel des Riegels 9 bzw. der daran ausgebildeten zweiten Koppelstruktur 11, entspricht im Wesentlichen dem Abstand von nach außen gewandten Flanken der U-Schenkel des Rahmens 4 auf Höhe des Spalts 8. Alternativ können der Riegel und der Rahmen 4 auch derart angepasst werden, dass die zweite Kopplungsstruktur außen an dem Riegel und die erste Kopplungsstruktur innen an dem Rahmen 4 vorgesehen ist.

Die freien Enden der U-Form des Riegels 9 haben schräge Flächen 12 bzw. sind nach innen hin derart abgeschrägt, dass der Abstand zwischen den inneren Flanken der U-Schenkel des Riegels 9 entlang der schrägen Flächen 12 nach außen hin zunimmt. Ferner ist an einer in y-Richtung gewandten Flanke des Riegels 9 (Außenkante), in dieser Ausführungsform auf Höhe des Basisabschnitts, eine Tasche (Einkerbung) 13 ausgebildet, die je einen Anschlag in positiver x-Richtung und einen Anschlag in negativer x-Richtung bereitstellt. Die durch die Tasche 13 ausgebildeten Anschläge sind dazu vorgesehen, mit zugehörigen, am zweiten Gehäuseteil 6 beispielsweise durch einen Vorsprung ausgebildeten Anschlägen in Anlage zu kommen und dadurch eine Bewegung des Riegels 9 in x-Richtung bezüglich des zweiten Gehäuseteils 6 zu begrenzen.

In einer zu der x-y-Ebene parallel verlaufenden Oberfläche der U-Basis des Riegels 9 ist eine sich in z-Richtung erstreckende Öffnung (Durchbruch) ausgebildet, welche als Führungsnut/Kulisse 14 dient. Die Führungsnut 14 verläuft in der Riegeloberfläche entlang einer S-Kurve. D.h. ein erstes Ende der Führungsnut liegt näher an einem Außenrand der U-Basis des Riegels 9 und verläuft zunächst parallel zu dem Außenrand der U-Basis (in y-Richtung), folgt anschließend einer Kurve weg von dem Außenrand der U-Basis und folgt dann einer Kurve in entgegengesetzter Richtung, sodass die Führungsnut 14 abschließend an ihrem zweiten Ende erneut in y-Richtung verläuft, dabei jedoch einen größeren Abstand zu dem Außenrand der U-Basis als an ihrem ersten Ende hat. Grundsätzlich kann die Führungsnut 14 in y-Richtung an jeder Stelle der U-Basis des Riegels 9 positioniert sein, vorteilhaft ist es jedoch, wenn sie wie in der vorliegenden Ausführungsform mittig angeordnet ist, um ein Verklemmen zu vermeiden.

Die Oberfläche der U-Basis des Riegels 9 sowie eine dazu entgegengesetzte, untere Fläche der U-Basis haben eine auffällige farbige Markierung 15 bzw. sind vorteilhafterweise vollständig eingefärbt. Alternativ kann auch der gesamte Riegel eingefärbt sein. Wie später noch genauer erläutert, steht der Riegel in seiner Entriegelungsstellung aus einem Schlitz 16 in dem zweiten Gehäuseteil 6 hervor, wodurch die Markierung 15 für Bedienpersonal auffällig sichtbar ist. Ein einfacher Hinweis, wie beispielsweise durch einen Zeiger, welcher auf ein "offen"- oder "geschlossen"-Symbol zeigt, könnte eine derartige Signalwirkung nicht erfüllen, da hierbei lediglich eine Stellung eines in jedem Zustand gleich aussehenden Bauteils sich ändert. Die nur im offenen Zustand freigelegt, auffällige, in Signalfarben gekennzeichnete Markierung 15 dagegen ist nicht nur ein einfacher Hinweis, sondern stellt zusätzlich einen Aufmerksamkeit erregenden Warnhinweis dar, welcher von Bedienpersonal nur schwer zu übersehen ist, selbst falls dieses nicht gesondert darauf achtet.

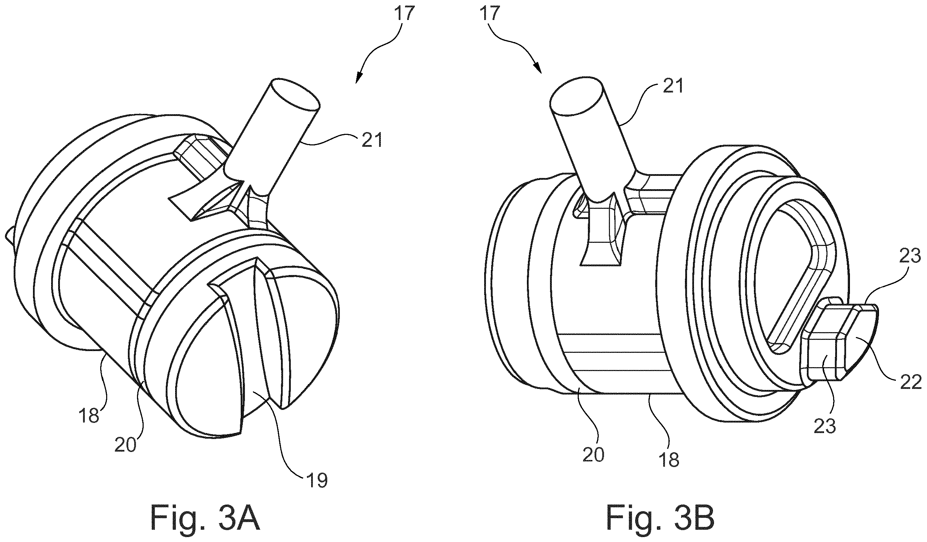

Fig. 3 zeigt eine perspektivische Ansicht eines Riegelschlosses 17 (Fig. 3A, links) und eine zweite perspektivische Ansicht des Riegelschlosses 17 von einer anderen Seite (Fig. 3B, rechts). Im montierten Zustand ist das Riegelschloss 17 drehbar in dem zweiten Gehäuseteil 6 gelagert. Wie in Fig. 3A gezeigt, hat das Riegelschloss 17 einen zylinderförmigen, Schlosskörper 18 an dessen Vorderseite eine Werkzeugaufnahme 19 zur Betätigung des Riegelschlosses 17 durch Bedienpersonal vorgesehen ist. Die Werkzeugaufnahme 19 ist in diese Ausführungsform ein mittels einer Münze oder Ähnlichem betätigbarer Schlitz, sie kann jedoch alternativ für jede Art von Werkzeug wie z.B. Schraubendreher angepasst sein. Weiter alternativ kann das Schloss anstelle einer Werkzeugaufnahme einen Griff aufweisen, welcher von Hand betätigbar ist. Vorteilhaft an einer Ausführungsform mit einer einfachen Werkzeugaufnahme ist, dass das Schloss nicht nach außen hin vorsteht und man somit nicht daran hängen bleiben und sich dadurch verletzen, die Vorrichtung umwerfen oder das Schloss dadurch möglicherweise sogar versehentlich betätigen kann.

An einer Mantelfläche des Schlosskörpers 18, vorzugsweise unmittelbar hinter der Vorderseite mit der Werkzeugaufnahme 19, ist eine Nut mit einem darin eingesetzten O-Ring 20 vorgesehen. Der O-Ring 20 dient zum einen zur Abdichtung zwischen dem Riegelschloss 17 und dem zweiten Gehäuseteil 6 und zum anderen erzeugt er einen erhöhten Reibungswiderstand dazwischen, wodurch das Riegelschloss 17 in seiner Position relativ zu dem zweiten Gehäuseteil 6 festgehalten wird, wenn es nicht entgegen dem Reibungswiderstand betätigt wird.

Hinter der Nut mit dem O-Ring 20 ist an der Mantelfläche des Schlosskörpers 18 ein Betätigungsstift 21 vorgesehen, welcher sich von der Mantelfläche aus in einer Radialrichtung des Schlosskörpers 18 nach außen erstreckt. Wenn das Riegelschloss 17 und der Riegel 9 in dem zweiten Gehäuseteil 6 eingesetzt sind, greift der Betätigungsstift 21 in die Führungsnut 14 des Riegels 9 ein. Wird das Riegelschloss 17 gedreht, gleitet der Betätigungsstift 21 entlang von Flanken der S-förmigen Führungsnut 14, wodurch die Drehbewegung des Riegelschlosses 17 in eine Linearbewegung des Riegels 9 gewandelt wird.

Wie in Fig. 3B gezeigt, ist auf einer der Werkzeugaufnahme 19 entgegengesetzten Rückseite des Schlosskörpers 18 ein exzentrisch angeordneter Nippel/Zapfen 22 ausgebildet, welcher bei einer Draufsicht entlang einer Mittelachse des Schlosskörpers 18 eine im Wesentlichen trapezähnliche Form mit zwei Anschlagsflächen 23 aufweist. Alternativ kann der Nippel 22 beispielsweise auch in Form eines Kreisausschnitts oder dreieckig ausgebildet sein. Die Ebenen, in welchen die beiden Anschlagsflächen 23 liegen, sind bezüglich der Zylindergrundfläche des Schlosskörpers 18 nach innen gewandt angeordnet, während eine ggf. gewölbte Grundseite der trapezähnlichen Form nach außen hin gewandt ist. Die Ebenen der Anschlagsflächen 23 können sich in der Mittelachse des Schlosskörpers 18 schneiden, sie können jedoch auch davon abweichende Winkel zueinander haben. Die Anschlagsflächen 23 des Riegelschlosses 17 sind dazu angepasst, mit zugehörigen Anschlagsflächen 24 des zweiten Gehäuseteils 6 in Anlage zu kommen und auf diese Weise eine Drehbewegung des Riegelschlosses 17 auf einen bestimmten Winkel, beispielsweise zwischen 70° und 100°, vorteilhafterweise 85° bis 90°, zu begrenzen. Dies verhindert, dass der Betätigungsstift 21 aus der Führungsnut 14 des Riegels 9 rutschen kann. Dieser Effekt wird auch durch die durch die in Fig. 2 beschriebene Tasche 13 an dem Riegel 9 bereitgestellten Anschläge erzielt, wodurch zum einen eine doppelte Absicherung gegen ein Herausrutschen des Betätigungsstifts 21 aus der Führungsnut 14 gewährleistet ist und zum anderen eine ggf. auf den Riegel 9 aufgebrachte Last, z.B. ein durch ein Anstoßen an dem geöffneten und aus dem zweiten Gehäuseteil 6 vorstehenden Riegel 9 aufgebrachter Schlag, nicht durch den empfindlicheren Betätigungsstift 21 sondern durch die die Anschläge des aufgrund seiner Geometrie und Materialauswahl stabileren Riegels 9 aufgenommen wird.

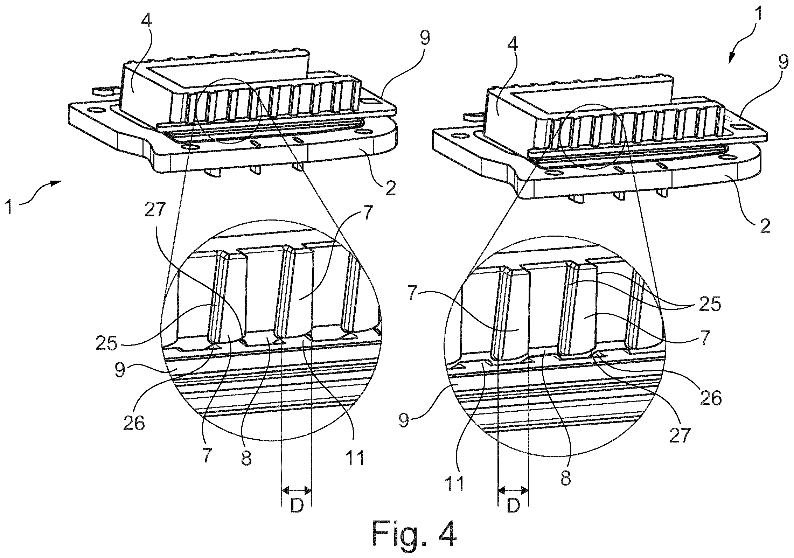

Fig. 4 zeigt eine Stellung des Riegels 9 und des ersten Gehäuseteils 1 zueinander in der Verriegelungsstellung (links) und in der Entriegelungsstellung (rechts) mit vergrößerten Detailansichten der Koppelstrukturen 7, 11. Dabei liegt der Riegel 9 in dem Spalt 8 des ersten Gehäuseteils 1. Es sollen insbesondere die erste und zweite Koppelstruktur 7, 11 anhand dieser Fig. 4 genauer erläutert werden.

Es ist deutlich zu erkennen, dass Flanken 25 der einzelnen ersten Vorsprünge oder Rippen der ersten Koppelstruktur 7 keilförmig sind. D.h., die einzelnen ersten Vorsprünge verlaufen nicht exakt senkrecht nach oben, in z-Richtung, sondern haben einen kleinen Winkel zueinander, vorzugsweise zwischen 3° und 30°, sodass sich zwischen den ersten Vorsprüngen befindliche Lücken nach oben hin leicht aufweiten. Bei Aufeinandersetzen der beiden Gehäuseteile 1, 6 schieben sich die zweiten Vorsprünge der an dem Riegel 9 angeordneten, zu der ersten Koppelstruktur 7 komplementären zweiten Koppelstruktur 11 durch die Lücken zwischen den ersten Vorsprüngen bis in den Spalt 8 zwischen der ersten Koppelstruktur 7 und der Grundplatte 2, wobei die zweiten Vorsprünge ggf. an den Flanken 25 der ersten Vorsprünge entlang gleiten. Bei diesem Vorgang dienen somit die leicht schräg verlaufenden Flanken 25 der ersten Vorsprünge als Zentrierungshilfen oder Einführhilfen für die zweiten Vorsprünge der zweiten Koppelstruktur 11 des Riegels 9.

Ferner ist hier gut zu erkennen, dass an dem unteren Ende der ersten Vorsprünge, d.h. an deren dem Spalt 8 zugewandten Seite, eine Breite der ersten Vorsprünge in x-Richtung im Wesentlichen dem Abstand zwischen zweien der ersten Vorsprünge, d.h. der Weite der Lücke dazwischen, entspricht, was wiederrum dem Raster bzw. Rastermaß entspricht. Da das Raster ferner, wie in Fig. 4 dargestellt, die Breite der zweiten Vorsprünge der zweiten Koppelstruktur 11 des Riegels 9 sowie eine Weite von Lücken zwischen zweien der zweiten Vorsprünge bestimmt, ist gut ersichtlich, dass die zweiten Vorsprünge des Riegels 9 während eines Verriegelungsprozesses zwischen die ersten Vorsprünge der ersten Koppelstruktur 7 gleiten, der Riegel 9 in dem Spalt 8 zu liegen kommt (Entriegelungsstellung, rechts) und darin in der Verriegelungsrichtung gegen die erste Koppelstruktur 7 verschoben wird, sodass in einer Verriegelungsstellung (links) die zweiten Vorsprünge unter den ersten Vorsprüngen passend positioniert sind bzw. in diese eingreifen und somit eine Bewegung der beiden Gehäuseteile 1, 6 in z-Richtung voneinander weg verhindern. Aufgrund der gemäß dem Raster über nahezu die gesamte Breite der Gehäuseteile 1, 6 hinweg verteilten Auflage- oder Eingriffsfläche zwischen den beiden Koppelstrukturen 7, 11 ermöglicht diese Verriegelung eine hohe Stabilität, Widerstandsfähigkeit und Zuverlässigkeit der Verbindung zwischen den Ordnungsmodulen O1, 02 des Ordnungssystems O.

Ferner zeigt Fig. 4, dass die zweiten Vorsprünge der zweiten Koppelstruktur 11 des Riegels 9 an einer der erstem Koppelstruktur 7 zugewandten Seite (Oberseite) in die Verriegelungsrichtung weisende (d.h., hin zu den freien Enden der U-Schenkel des Riegels 9 gewandten) zweite Rampen 26 vorgesehen. Gleichermaßen sind an einer der zweiten Koppelstruktur 11 bzw. dem Spalt 8 zugewandten Seite (Unterseite) der ersten Vorsprünge in die Entriegelungsrichtung weisende (d.h., hin zu den freien Enden der U-Schenkel des Rahmens 4 und weg von den freien Enden der U-Schenkel des Riegels 9 gewandte) erste Rampen 27 vorgesehen. Auf diese Weise treffen bei einem Verriegelungsvorgang die zweiten Rampen 26 der zweiten Vorsprünge des Riegels 9 auf die ersten Rampen 27 der ersten Vorsprünge des Rahmens 4, gleiten an diesen ab und tragen auf diese Weise dazu bei, dass der Riegel 9 sich nicht verkantet, sondern sich durch das Abgleiten unter die ersten Vorsprünge schiebt. Wenn der Riegel 9 auf diese Weise unter die ersten Vorsprünge geschoben wird, wird ferner aufgrund der Rampen 26, 27 eine in z-Richtung wirkende Kraft zwischen den beiden Gehäuseteilen 1, 6 erzeugt, durch welche diese zusammengezogen werden. Dies trägt dazu bei, die Daten- und/oder Energieübertragungseinheiten 5 zuverlässig zu verbinden.

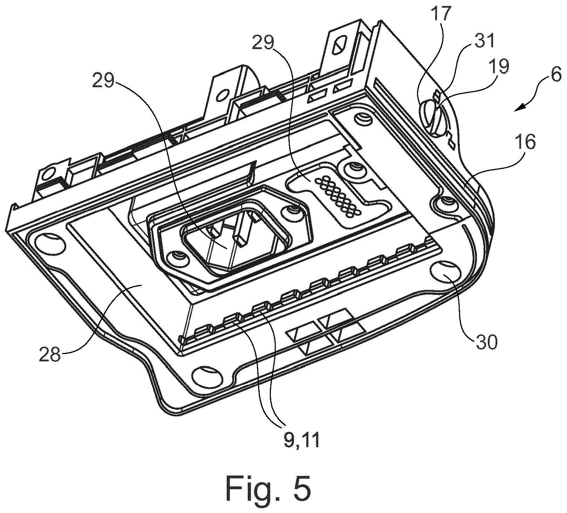

Fig. 5 zeigt eine perspektivische Ansicht des zweiten Gehäuseteils 6 von unten, gemäß der Ausführungsform, welcher in diesem Ausführungsbeispiel ein unterer Gehäuseteil oder Boden ist. Grundsätzlich kann er jedoch auch ein oberer Gehäuseteil sein. Es ist gut erkennbar, dass in diesem zweiten Gehäuseteil 6 eine Mulde 28 ausgebildet ist, welche die als Zentrierungshilfen dienenden Innenflächen des zweiten Gehäuseteils 6 bereitstellt und zur Aufnahme des Rahmens 4 dient. Ferner sind zu den Daten- und/oder Energieübertragungseinheiten 5 des ersten Gehäuseteils 1 komplementäre Daten- und/oder Energieübertragungseinheiten 29 in der Mulde 28 angeordnet. Außerhalb der Mulde 28 sind Durchgangslöcher 30 an dem zweiten Gehäuseteil 6 vorgesehen, welche zur Befestigung desselben, beispielsweise durch Schrauben, an dem zweiten Ordnungsmodul O2 dienen.

In dem zweiten Gehäuseteil 6 ist der Riegel 9 in x-Richtung verschiebbar gelagert und ist das Riegelschloss 17 drehbar gelagert. Das Riegelschloss 17 ist dabei mit seiner Werkzeugaufnahme 19 an einer Seite des zweiten Gehäuseteils 6 für Bedienpersonal zugänglich exponiert. Zudem ist in der Seite des zweiten Gehäuseteils 6 neben der Werkzeugaufnahme 19 eine Beschriftung 31 vorgesehen, welche je nach Winkelposition der schlitzförmigen Werkzeugaufnahme 19 eine Ver- oder Entriegelungsstellung des Riegelschlosses 17 anzeigt. Darüber hinaus ist in der Seite des zweiten Gehäuseteils 6 neben der Werkzeugaufnahme 19 der Schlitz 16 vorgesehen, in welchen die U-Basis des Riegels 9 mit der Markierung 15 in der in Fig. 5 dargestellten Verriegelungsstellung versenkt ist und in der Entriegelungsposition auffällig hervorsteht.

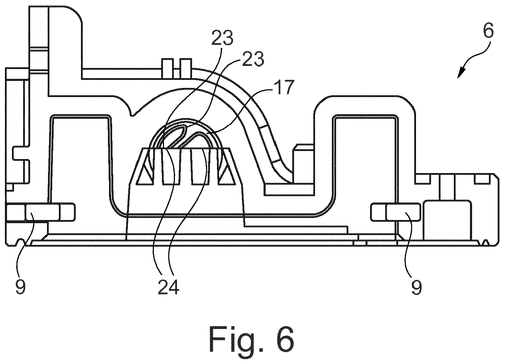

Fig. 6 zeigt einen Schnitt durch den zweiten Gehäuseteil 6, in welchem der Riegel 9 und das Riegelschloss 17 eingebaut sind, entlang einer Schnittebene, welche derart senkrecht zu der x-Richtung verläuft, um eine unmittelbare Draufsicht von innerhalb der Mulde 28 auf das Riegelschloss 17 zu ermöglichen. In dieser Ansicht ist erkennbar, dass einer der Anschlagsflächen 23 des Nippels 22 an einer der zugehörigen Anschlagsflächen 24 des zweiten Gehäuseteils 6 in Anlage ist und das Riegelschloss sich somit in einer Endstellung befindet, d.h. der Riegel 9 entweder vollständig verriegelt oder entriegelt ist.

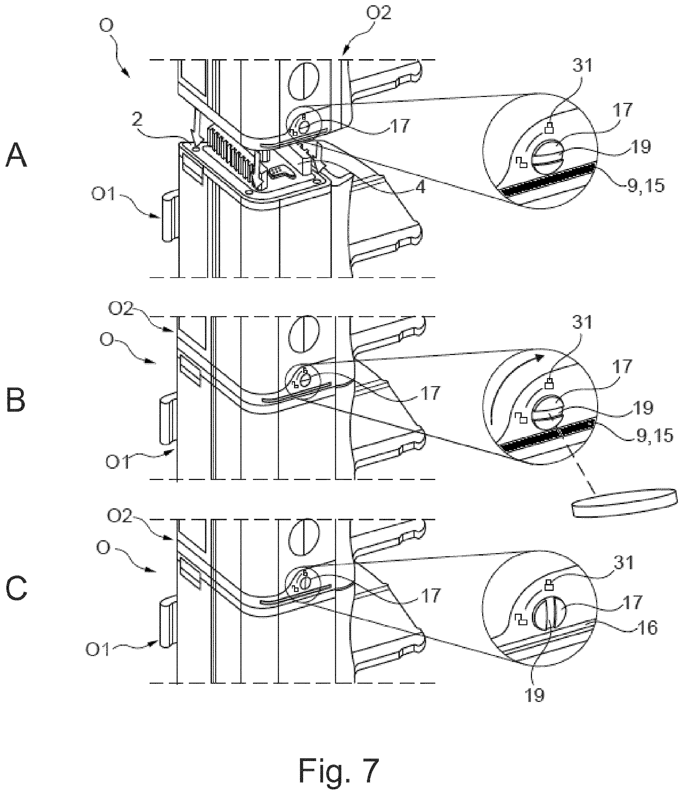

Fig. 7 veranschaulicht einen Montagevorgang der beiden Ordnungsmodule O1, O2 gemäß der Ausführungsform. Fig. 7A (oben) zeigt das erste (untere) und das zweite (obere) Ordnungsmodul O1, O2, welche, wie durch Pfeile veranschaulicht, gerade aufeinandergesetzt werden. Zunächst ist das Riegelschloss 17, wie im dem vergrößerten Ausschnitt rechts hervorgehoben, in eine durch die Beschriftung 31 gekennzeichnete Entriegelungsstellung gedreht. Dabei steht der Riegel 9 mit der Markierung 15 (in der Fig. Schwarz gefüllte Kontur) aus dem Schlitz 16 hervor und macht so auf auffällige Weise den entriegelten Zustand aufmerksam. Während des Aufeinandersetzens werden die Kopplungsstrukturen 7, 11 wie vorstehend beschrieben ineinander geschoben.

In Fig. 7B (mittig) sind die beiden Ordnungsmodule 01, 02 aufeinandergesetzt und zentriert, sodass sie bündig miteinander abschließen, sind jedoch noch nicht verriegelt, wie durch die Beschriftung 31 und die Markierung 15 angezeigt. Entsprechend können sie noch in x-Richtung voneinander wegbewegt und getrennt werden. Der Riegel 9 liegt in dieser Position in dem Spalt 8, die zweiten Vorsprünge der zweiten Koppelstruktur 11 liegen jedoch nicht unter den ersten Vorsprüngen der ersten Koppelstruktur 7, sondern sind dazu versetzt angeordnet und überlappen diese nicht. Um die Ordnungsmodule O1, O2 zu verriegeln wird ein beispielhaft als Münze dargestelltes Werkezeug in die Werkzeugaufnahme 19 eingesetzt, um das Riegelschloss 17 und dadurch den Riegel 9 zu betätigen.

Fig. 7C (unten) zeigt den verriegelten Zustand der beiden Ordnungsmodule O1, O2, welcher dadurch gekennzeichnet ist, dass zum einen die Werkzeugaufnahme 19 des Riegelschlosses 17 auf die entsprechende Beschriftung verweist und insbesondere die U-Basis des Riegels 9, welche die Markierung 15 aufweist, nicht aus dem Schlitz 16 hervorsteht und somit kein Warnsignal ausgibt.

### Liste der Referenzzeichen

- 1: erster Gehäuseteil
- 2: Grundplatte des ersten Gehäuseteils
- 3: Durchgangslöcher des ersten Gehäuseteils
- 4: Rahmen des ersten Gehäuseteils
- 5: Daten- und/oder Energieübertragungseinheiten des ersten Gehäuseteils
- 6: zweiter Gehäuseteil
- 7: erste Koppelstruktur (erste Vorsprünge/Rippen) des ersten Gehäuseteils
- 8: Spalt des ersten Gehäuseteils
- 9: Riegel
- 10: Erhebung des ersten Gehäuseteils
- 11: Zweite Koppelstruktur (zweite Vorsprünge/Rippen) des Riegels
- 12: Schräge Flächen des Riegels
- 13: Tasche (Anschläge) des Riegels
- 14: Führungsnut des Riegels
- 15: Markierung des Riegels
- 16: Schlitz (Öffnung) des zweiten Gehäuseteils
- 17: Riegelschloss
- 18: Schlosskörper des Riegelschlosses
- 19: Werkzeugaufnahme des Riegelschlosses
- 20: O-Ring des Riegelschlosses
- 21: Betätigungsstift des Riegelschlosses
- 22: Nippel des Riegelschlosses
- 23: Anschlagsflächen des Nippels des Riegelschlosses
- 24: Anschlagsflächen des zweiten Gehäuseteils
- 25: Flanken der ersten Vorsprünge des ersten Gehäuseteils
- 26: zweite Rampe der zweiten Vorsprünge (Koppelstruktur) des Riegels
- 27: erste Rampe der ersten Vorsprünge (Koppelstruktur) des Rahmens
- 28: Mulde des zweiten Gehäuseteils
- 29: Daten- und/oder Energieübertragungseinheiten des zweiten Gehäuseteils
- 30: Durchgangslöcher des zweiten Gehäuseteils
- 31: Beschriftung des zweiten Gehäuseteils
- O: modulares Ordnungssystem
- O1: erstes Ordnungsmodul
- O2: zweites Ordnungsmodul
- D: bestimmte Distanz / Verschiebedistanz des Riegels

## Patentansprüche

1. Modulares Ordnungssystem (O) zum Ordnen medizinischer Geräte, insbesondere Infusionspumpen, mit zumindest zwei mechanisch miteinander verbindbaren Ordnungsmodulen (O1, O2),
wobei ein erstes Ordnungsmodul (O1) einen ersten Gehäuseteil (1) hat, an welchem an zumindest zwei einander diametral gegenüberliegenden Seiten eine erste Koppelstruktur (7) vorgesehen ist,
wobei ein zweites Ordnungsmodul (O2) einen zweiten Gehäuseteil (6) hat, welcher dafür angepasst ist, auf, unter oder seitlich an den ersten Gehäuseteil (1) des ersten Ordnungsmoduls (O1) montiert zu werden, und welcher einen drehbar oder verschiebbar in dem zweiten Gehäuseteil (6) gelagerten Riegel (9) zum Verriegeln der beiden Gehäuseteile (1, 6) aufweist, und
wobei jedes der zumindest zwei Ordnungsmodule (O1, O2) sowohl den ersten als auch den zweiten Gehäuseteil (1, 6) aufweist, und jedes Ordnungsmodul (O1, O2) mit jedem beliebigen weiteren Ordnungsmodul (O1, O2) koppelbar ist
**dadurch gekennzeichnet, dass**
die erste Koppelstruktur (7) ein Raster von ersten Vorsprüngen aufweist, der Riegel eine zweite Koppelstruktur (11) mit einem komplementären Raster von zweiten Vorsprüngen aufweist, welche dafür angepasst sind, in die ersten Vorsprünge hinterschneidend einzugreifen, und der Riegel (9) derart gestaltet ist, dass er durch eine singuläre Betätigung in die erste Koppelstruktur (7) an zumindest zwei einander diametral gegenüberliegenden Seiten des ersten Gehäuseteils (1) des ersten Ordnungsmoduls (O1) vorzugsweise gleichzeitig hinterschneidend eingreift.

2. Modulares Ordnungssystem (O) nach Anspruch 1, wobei der vorzugsweise U-förmige Riegel (9) linear um eine bestimmte Distanz (D) verschiebbar ist und wobei das Raster der ersten und zweiten Vorsprünge die bestimmte Distanz (D) festlegt.

3. Modulares Ordnungssystem (O) nach einem der Ansprüche 1 oder 2, wobei
die ersten Vorsprünge der ersten Koppelstruktur (7) des Rahmens (4) in eine Entriegelungsrichtung des Riegels (9) gewandte erste Rampen (27) aufweisen, und/oder
die zweiten Vorsprünge der zweiten Koppelstruktur (11) des Riegels (9) in eine Verriegelungsrichtung des Riegels (9) gewandte zweite Rampen (26) aufweisen.

4. Modulares Ordnungssystem (O) nach einem der Ansprüche 1 bis 3, wobei der Riegel (9) in dessen Verriegelungsrichtung und/oder Entriegelungsrichtung einen bzw. jeweils einen Anschlag (13) aufweist, welcher dazu angepasst ist, um mit einem zugehörigen Anschlag des zweiten Gehäuseteils (6) in Anlage zu kommen.

5. Modulares Ordnungssystem (O) nach einem der Ansprüche 1 bis 4, wobei ein eine Markierung (15) aufweisender Teil des Riegels (9) freiliegt, wenn die zwei Ordnungsmodule (O1, O2) sich in einem unverriegelten Zustand befinden, um ein auffälliges visuelles Feedback bezüglich des Zustands bereitzustellen

6. Modulares Ordnungssystem (O) nach Anspruch 5, wobei
ein die Markierung (15) aufweisender Teil des Riegels (9) aus einer Öffnung (16) des zweiten Gehäuseteils (6) hervorsteht, wenn die zwei Ordnungsmodule (O1, O2) sich in einem unverriegelten Zustand befinden, und
der die Markierung (15) aufweisende Teil des Riegels (9) durch das zweite Gehäuseteil (6) bedeckt ist, wenn die zwei Ordnungsmodule (O1, O2) sich in einem verriegelten Zustand befinden.

7. Modulares Ordnungssystem (O) nach einem der Ansprüche 1 bis 6, wobei ein drehbar in dem zweiten Gehäuseteil gelagertes, freiliegendes Riegelschloss (17) zum Betätigen des Riegels (9) vorgesehen ist.

8. Modulares Ordnungssystem (O) nach Anspruch 7, wobei das Riegelschloss (17) in einer Drehrichtung des Riegelschlosses (17) jeweils einen vorwärts und/oder rückwärts gewandten Anschlag (23) aufweist, welcher dazu angepasst ist, um mit einem zugehörigen Anschlag (24) des zweiten Gehäuseteils (6) in Anlage zu kommen.

9. Modulares Ordnungssystem (O) nach Anspruch 7 oder 8, wobei das Riegelschloss (17) in eine Führungsnut (14) des Riegels (9) eingreift und die Führungsnut (14) derart geformt ist, dass eine Drehbewegung des Riegelschlosses (17) über diese Führungsnut (14) in eine Linearbewegung des Riegels (9) gewandelt wird.

10. Modulares Ordnungssystem (O) nach einem der Ansprüche 7 bis 9, wobei ein eine Markierung (15) aufweisender Teil des Riegelschlosses (17) freiliegt, wenn die zwei Ordnungsmodule (O1, O2) sich in einem unverriegelten Zustand befinden, um ein auffälliges visuelles Feedback bezüglich des Zustands bereitzustellen.

11. Medizinisches Gerät, insbesondere Infusionspumpe mit einem Gehäuse, welches das modulare Ordnungssystem (O) nach einem der vorstehenden Ansprüche ausbildet, wobei vorzugsweise das erste Ordnungsmodul (O1) ein Gehäusedeckel und das zweite Ordnungsmodul (O2) ein Gehäuseboden ist.

## Claims

1. A modular arrangement system (O) for arranging medical devices, in particular infusion pumps, the modular arrangement system comprising at least two arrangement modules (O1, O2) which can be mechanically connected to one another,
wherein a first arrangement module (O1) has a first housing part (1) on which a first coupling structure (7) is provided on at least two diametrically opposed sides,
wherein a second arrangement module (O2) has a second housing part (6) which is adapted to be mounted on, below or laterally to the first housing part (1) of the first arrangement module (O1) and comprises a bar (9) rotatably or slidably supported in the second housing part (6) for locking the two housing parts (1, 6), and
wherein each of the at least two arrangement modules (O1, O2) has both the first and the second housing part (1, 6), and each arrangement module (O1, O2) can be coupled to any further arrangement module (O1, O2),
**characterized in that**
the first coupling structure (7) comprises a raster of first protrusions, the bar comprises a second coupling structure (11) having a complementary raster of second protrusions adapted to engage with the first protrusions in an undercutting manner, and the bar (9) is designed in such a way that, by a singular actuation, it engages with the first coupling structure (7) on at least two diametrically opposite sides of the first housing part (1) of the first arrangement module (O1), preferably simultaneously, in an undercutting manner.

2. The modular arrangement system (O) according to claim 1, wherein the preferably U-shaped bar (9) can be linearly displaced by a defined distance (D), and wherein the raster of the first and second protrusions sets the defined distance (D).

3. The modular arrangement system (O) according to any of claims 1 or 2, wherein
the first protrusions of the first coupling structure (7) of the frame (4) have first ramps (27) facing an unlocking direction of the bar (9), and/or
the second protrusions of the second coupling structure (11) of the bar (9) have second ramps (26) facing a locking direction of the bar (9).

4. The modular arrangement system (O) according to any of claims 1 to 3, wherein the bar (9) in its locking direction and/or unlocking direction has one or respectively one stop (13) adapted to come into contact with an associated stop of the second housing part (6).

5. The modular arrangement system (O) according to any of claims 1 to 4, wherein a portion of the bar (9) having a marking (15) is exposed when the two arrangement modules (O1, O2) are in an unlocked state to provide conspicuous visual feedback regarding the state.

6. The modular arrangement system (O) according to claim 5, wherein
a portion of the bar (9) having the marking (15) projects from an opening (16) of the second housing part (6) when the two arrangement modules (O1, O2) are in an unlocked state, and
the portion of the bar (9) having the marking (15) is covered by the second housing part (6) when the two arrangement modules (O1, O2) are in a locked state.

7. The modular arrangement system (O) according to any of claims 1 to 6, wherein an exposed bar lock (17) rotatably mounted in the second housing part is provided for actuating the bar (9).

8. The modular arrangement system (O) according to claim 7, wherein the bar lock (17), in a direction of rotation of the bar lock (17), comprises a respectively forwardly and/or rearwardly facing stop (23) adapted to come into contact with an associated stop (24) of the second housing part (6).

9. The modular arrangement system (O) according to claim 7 or 8, wherein the bar lock (17) engages in a guiding groove (14) of the bar (9) and the guiding groove (14) is shaped such that a rotational movement of the bar lock (17) is converted via this guiding groove (14) into a linear movement of the bar (9).

10. The modular arrangement system (O) according to any of claims 7 to 9, wherein a portion of the bar lock (17) having a marking (15) is exposed when the two arrangement modules (O1, O2) are in an unlocked state to provide conspicuous visual feedback regarding the state.

11. A medical device, in particular an infusion pump having a housing which forms the modular arrangement system (O) according to any of the preceding claims, wherein preferably the first arrangement module (O1) is a housing cover and the second arrangement (O2) module is a housing base.

## Revendications

1. Système de rangement modulaire (O) pour le rangement d'appareils médicaux, en particulier de pompes à perfusion, avec au moins deux modules de rangement (O1, 02) pouvant être reliés mécaniquement entre eux,
dans lequel un premier module de rangement (O1) présente une première partie de boîtier (1) au niveau de laquelle une première structure de couplage (7) est prévue sur au moins deux côtés diamétralement opposés,
dans lequel un second module de rangement (02) présente une seconde partie de boîtier (6) qui est adaptée pour être montée sur, sous ou latéralement par rapport à la première partie de boîtier (1) du premier module de rangement (O1) et qui présente un verrou (9) monté de manière rotative ou déplaçable dans la seconde partie de boîtier (6) pour verrouiller les deux parties de boîtier (1, 6), et
dans lequel chacun des au moins deux modules de rangement (O1, 02) présente à la fois la première et la seconde partie de boîtier (1, 6), et chaque module de rangement (O1, 02) peut être couplé à un quelconque module de commande (O1, 02) supplémentaire
**caractérisé en ce que**
la première structure de couplage (7) présente un réseau de premières saillies, le verrou présente une seconde structure de couplage (11) avec un réseau complémentaire de secondes saillies qui sont adaptées pour s'engager en contre-dépouille dans les premières saillies, et le verrou (9) est conçu de sorte qu'il s'engage en contre-dépouille, de préférence simultanément, dans la première structure de couplage (7) par un seul actionnement au niveau d'au moins deux côtés diamétralement opposés de la première partie de boîtier (1) du premier module de rangement (O1).

2. Système de rangement modulaire (O) selon la revendication 1, dans lequel le verrou (9), de préférence en forme de U, est déplaçable linéairement d'une certaine distance (D) et dans lequel le réseau des premières et secondes saillies détermine la certaine distance (D).

3. Système de rangement modulaire (O) selon l'une quelconque des revendications 1 ou 2, dans lequel
les premières saillies de la première structure de couplage (7) du cadre (4) présentent de premières rampes (27) orientées dans une direction de déverrouillage du verrou (9), et/ou
les secondes saillies de la seconde structure de couplage (11) du verrou (9) présentent de secondes rampes (26) orientées dans une direction de verrouillage du verrou (9).

4. Système de rangement modulaire (O) selon l'une quelconque des revendications 1 à 3, dans lequel le verrou (9) présente une ou respectivement une butée (13) dans sa direction de verrouillage et/ou sa direction de déverrouillage, butée qui est adaptée pour venir en appui sur une butée associée de la seconde partie de boîtier (6).

5. Système de rangement modulaire (O) selon l'une quelconque des revendications 1 à 4, dans lequel une partie du verrou (9) présentant un repère (15) est exposée lorsque les deux modules de rangement (O1, 02) sont dans un état déverrouillé, pour fournir un retour visuel frappant concernant l'état.

6. Système de rangement modulaire (O) selon la revendication 5, dans lequel
une partie du verrou (9) présentant le repère (15) dépasse d'une ouverture (16) de la seconde partie de boîtier (6) lorsque les deux modules de rangement (O1, 02) se trouvent dans un état déverrouillé, et
la partie du verrou (9) présentant le repère (15) est recouverte par la seconde partie de boîtier (6) lorsque les deux modules de rangement (O1, 02) se trouvent dans un état verrouillé.

7. Système de rangement modulaire (O) selon l'une quelconque des revendications 1 à 6, dans lequel une serrure de verrou (17) exposée et montée de manière rotative dans la seconde partie de boîtier est prévue pour actionner le verrou (9).

8. Système de rangement modulaire (O) selon la revendication 7, dans lequel la serrure de verrou (17) présente, dans un sens de rotation de la serrure de verrou (17), respectivement une butée (23) tournée vers l'avant et/ou vers l'arrière qui est adaptée pour venir en appui sur une butée associée (24) de la seconde partie de boîtier (6).

9. Système de rangement modulaire (O) selon la revendication 7 ou 8, dans lequel la serrure de verrou (17) s'engage dans une rainure de guidage (14) du verrou (9) et la rainure de guidage (14) est formée de sorte qu'un mouvement de rotation de la serrure de verrou (17) soit transformé en un mouvement linéaire du verrou (9) par l'intermédiaire de cette rainure de guidage (14).

10. Système de rangement modulaire (O) selon l'une quelconque des revendications 7 à 9, dans lequel une partie de la serrure de verrou (17) présentant un repère (15) est exposée lorsque les deux modules de rangement (O1, 02) sont dans un état déverrouillé, pour fournir un retour visuel frappant concernant l'état.

11. Appareil médical, en particulier pompe à perfusion avec un boîtier, qui forme le système de rangement modulaire (O) selon l'une quelconque des revendications précédentes, dans lequel, de préférence, le premier module de rangement (O1) est un couvercle de boîtier et le second module de rangement (02) est un fond de boîtier.
